(19) Europäisches Patentamt
European Patent Office
Office européen des brevets

(11) EP 3 444 264 A1

(12) EUROPEAN PATENT APPLICATION
published in accordance with Art. 153(4) EPC

(43) Date of publication:
20.02.2019 Bulletin 2019/08

(21) Application number: 17782460.4

(22) Date of filing: 13.04.2017

(51) Int Cl.:
C07K 5/11 (2006.01)   A61K 38/07 (2006.01)
A61P 25/28 (2006.01)   C07K 5/00 (2006.01)
C07K 5/117 (2006.01)   C12N 15/00 (2006.01)
C12N 15/09 (2006.01)   C12Q 1/02 (2006.01)
G01N 33/15 (2006.01)   G01N 33/50 (2006.01)

(86) International application number:
PCT/JP2017/015090

(87) International publication number:
WO 2017/179647 (19.10.2017 Gazette 2017/42)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 14.04.2016 JP 2016081031

(71) Applicant: TAO Health Life Pharma Co., Ltd.
Kyoto 606-8501 (JP)

(72) Inventors:
• HOSHI, Minako
Sakyo-ku, Kyoto-shi
Kyoto 606-8501 (JP)

• SASAHARA, Tomoya
Sakyo-ku, Kyoto-shi
Kyoto 606-8501 (JP)
• SAIJYO, Eri
Sakyo-ku, Kyoto-shi
Kyoto 606-8501 (JP)
• IWASAKI, Genji
Takasaki-shi
Gunma 370-0034 (JP)
• MORI, Ichiro
Ashiya-shi
Hyogo 659-0095 (JP)

(74) Representative: Dehns
St. Brides House
10 Salisbury Square
London EC4Y 8JD (GB)

(54) PEPTIDE FOR INHIBITING BINDING OF AMYLOSPHEROIDS (ASPD), AND EVALUATION AND SCREENING METHOD

(57) Provided is a peptide having an ability to inhibit binding between amylospheroids (ASPD) and neurons. In one aspect, provided is a synthesized, isolated, or purified polypeptide represented by Formula (I) or (II) below: $X_1X_2X_3X_4$ (I), where $X_1$ is R, H, or K, $X_2$ is R, K, D, H, W, Q, Y, or T, $X_3$ is any amino acid, and $X_4$ is W, F, or Y; and $X_1X_2X_3X_4$ (II), where $X_1$ is R, H, or K, $X_2$ is any amino acid, $X_3$ is L, G, I, W, R, M, D, E, A, V, or K, and $X_4$ is W, F, or Y.

EP 3 444 264 A1

FIG. 3

SEQUENCE LISTING

<110> TAO Health Life Pharma Co., Ltd.

<120> Peptide for Inhibition of Binding between ASPD and Neuron, Assessment, and Screening

<130> H4479-01

<150> JP2016-81031
<151> 2016-04-14

<160> 28

<170> PatentIn version 3.5

<210> 1
<211> 4
<212> PRT
<213> Artificial Sequence

<220>
<223> Synthetic Peptide

<220>
<221> MISC_FEATURE
<222> (1)..(1)
<223> Xaa=Arg, His, or Lys

<220>
<221> MISC_FEATURE
<222> (2)..(2)
<223> Xaa=Arg, Lys, Asp, His, Trp, Gln, Tyr, or Thr

<220>
<221> MISC_FEATURE
<222> (3)..(3)
<223> Xaa=any amino acid

<220>
<221> MISC_FEATURE
<222> (4)..(4)
<223> Xaa=Trp, Phe, or Tyr

<400> 1

Xaa Xaa Xaa Xaa
1

<210> 2
<211> 4
<212> PRT
<213> Artificial Sequence

<220>
<223> Synthetic Peptide

<220>
<221> MISC_FEATURE
<222> (1)..(1)

<223>    Xaa=Arg, His, or Lys

<220>
<221>    MISC_FEATURE
<222>    (2)..(2)
<223>    Xaa=any amino acid

<220>
<221>    MISC_FEATURE
<222>    (3)..(3)
<223>    Xaa=Leu, Gly, Ile, Trp, Arg, Met, Asp, Glu, Ala, Val, or Lys

<220>
<221>    MISC_FEATURE
<222>    (4)..(4)
<223>    Xaa=Trp, Phe, or Tyr

<400>    2

Xaa Xaa Xaa Xaa
1


<210>    3
<211>    4
<212>    PRT
<213>    Artificial Sequence

<220>
<223>    Synthetic Peptide

<400>    3

His Phe Asn Trp
1


<210>    4
<211>    4
<212>    PRT
<213>    Artificial Sequence

<220>
<223>    Synthetic Peptide

<400>    4

His Arg Asn Trp
1


<210>    5
<211>    4
<212>    PRT
<213>    Artificial Sequence

<220>
<223>    Synthetic Peptide

<400>    5

His Lys Asn Trp
1

```
<210>  6
<211>  4
<212>  PRT
<213>  Artificial Sequence

<220>
<223>  Synthetic Peptide

<400>  6

His Asp Asn Trp
1


<210>  7
<211>  4
<212>  PRT
<213>  Artificial Sequence

<220>
<223>  Synthetic Peptide

<400>  7

His His Asn Trp
1


<210>  8
<211>  4
<212>  PRT
<213>  Artificial Sequence

<220>
<223>  Synthetic Peptide

<400>  8

His Trp Asn Trp
1


<210>  9
<211>  4
<212>  PRT
<213>  Artificial Sequence

<220>
<223>  Synthetic Peptide

<400>  9

His Gln Asn Trp
1


<210>  10
<211>  4
<212>  PRT
<213>  Artificial Sequence
```

```
<220>
<223>   Synthetic Peptide

<400>   10

His Tyr Asn Trp
1


<210>   11
<211>   4
<212>   PRT
<213>   Artificial Sequence

<220>
<223>   Synthetic Peptide

<400>   11

His Thr Asn Trp
1


<210>   12
<211>   4
<212>   PRT
<213>   Artificial Sequence

<220>
<223>   Synthetic Peptide

<400>   12

His Ala Asn Trp
1


<210>   13
<211>   4
<212>   PRT
<213>   Artificial Sequence

<220>
<223>   Synthetic Peptide

<400>   13

His Val Asn Trp
1


<210>   14
<211>   4
<212>   PRT
<213>   Artificial Sequence

<220>
<223>   Synthetic Peptide

<400>   14

His Phe Leu Trp
1
```

```
<210>  15
<211>  4
<212>  PRT
<213>  Artificial Sequence

<220>
<223>  Synthetic Peptide

<400>  15

His Phe Gly Trp
1


<210>  16
<211>  4
<212>  PRT
<213>  Artificial Sequence

<220>
<223>  Synthetic Peptide

<400>  16

His Phe Ile Trp
1


<210>  17
<211>  4
<212>  PRT
<213>  Artificial Sequence

<220>
<223>  Synthetic Peptide

<400>  17

His Phe Trp Trp
1


<210>  18
<211>  4
<212>  PRT
<213>  Artificial Sequence

<220>
<223>  Synthetic Peptide

<400>  18

His Phe Arg Trp
1


<210>  19
<211>  4
<212>  PRT
<213>  Artificial Sequence
```

```
<220>
<223>   Synthetic Peptide

<400>   19

His Phe Met Trp
1


<210>   20
<211>   4
<212>   PRT
<213>   Artificial Sequence

<220>
<223>   Synthetic Peptide

<400>   20

His Phe Asp Trp
1


<210>   21
<211>   4
<212>   PRT
<213>   Artificial Sequence

<220>
<223>   Synthetic Peptide

<400>   21

His Phe Glu Trp
1


<210>   22
<211>   4
<212>   PRT
<213>   Artificial Sequence

<220>
<223>   Synthetic Peptide

<400>   22

His Phe Ala Trp
1


<210>   23
<211>   4
<212>   PRT
<213>   Artificial Sequence

<220>
<223>   Synthetic Peptide

<400>   23

His Phe Val Trp
1
```

```
<210>  24
<211>  4
<212>  PRT
<213>  Artificial Sequence

<220>
<223>  Synthetic Peptide

<400>  24

His Phe Lys Trp
1


<210>  25
<211>  4
<212>  PRT
<213>  Artificial Sequence

<220>
<223>  Synthetic Peptide

<400>  25

His Phe His Trp
1


<210>  26
<211>  4
<212>  PRT
<213>  Artificial Sequence

<220>
<223>  Synthetic Peptide

<400>  26

His Phe Tyr Trp
1


<210>  27
<211>  4
<212>  PRT
<213>  Artificial Sequence

<220>
<223>  Synthetic Peptide

<400>  27

His Asn Asn Trp
1


<210>  28
<211>  4
<212>  PRT
<213>  Artificial Sequence
```

```
<220>
<223>  Synthetic Peptide

<400>  28

His Pro Asn Trp
1
```

**Description**

[Technical Field]

The present disclosure relates to a peptide having an ability to inhibit binding between amylospheroids (ASPD) and neurons as well as a method of evaluating the same and a method of screening the same.

[Background Art]

Amylospheroids (ASPD; amylospheroids) each are a spherical Aβ assembly that is formed of about 30 amyloid β proteins (Aβ) aggregated together and has a diameter of approximately 10 nm, and are a structure considered to play an important role in the irreversible stage at which Alzheimer's disease develops.

ASPD were initially isolated as an in vitro synthesized Aβ assembly (i.e., synthetic ASPD) that exhibited strong neurotoxicity (Non-Patent Document 1). Antibodies specific to this synthetic ASPD have been produced (Patent Documents 1 and 2), and using these antibodies, ASPD formed in vivo (that is, native ASPD) were actually isolated from the brain of a human patient with Alzheimer's disease (Non-Patent Document 1).

Native ASPD and synthetic ASPD likewise selectively induce cell death of mature neurons. It was discovered that the target of ASPD in the neuronal cell death is the synaptic protein "alpha 3 subunit of $Na^+$, $K^+$-ATPase pump (hereinafter referred to as 'NAKα3')" that plays a very important role in neuronal survival and function, and it was revealed that the function of NAKα3 decreases due to the binding of ASPD and neurons are excited excessively, which results in death of neurons (Patent Document 3 and Non-Patent Document 2). Peptides capable of inhibiting the interaction between ASPD and NAKα3 have also been reported (Patent Document 3 and Non-Patent Document 2).

The most correlated with clinical symptoms in Alzheimer's disease is neuronal loss. It was revealed that the amount of native ASPD in the cerebral cortex of an Alzheimer's disease patient with neuronal loss increases relative to the severity of Alzheimer's disease and only a trace amount of native ASPD exists in the cerebellum of an Alzheimer's disease patient with little neuronal loss (Non-Patent Document 3). Therefore, ASPD are considered to play an important role in the irreversible stage at which Alzheimer's disease develops. Furthermore, native ASPD have also been detected from the brains of patients with Lewy body dementia (Non-Patent Document 3). Therefore, similarly in Lewy body dementia, ASPD are considered to play an important role in the development thereof.

Synthetic ASPD, which are considered to be equivalent to native ASPD, can be produced by slowly rotating a liquid containing Aβ (Non-Patent Document 1 and Patent Document 4).

[Prior Art Documents]

[Patent Documents]

[Patent Document 1] WO2006/016644
[Patent Document 2] WO2009/057664
[Patent Document 3] WO2013/099806
[Patent Document 4] WO2013/094614

[Non-Patent Documents]

[Non-Patent Document 1] Hoshi et al., Spherical aggregates of β-amyloid (amylospheroids) show high neurotoxicity and activate tau protein kinase I/glycogen synthase kinase-3β, PNAS May 27, 2003 vol. 100 no. 11 6370-6375
[Non-Patent Document 2] Ohnishi et al., Na, K-ATPase α3 is a death target of Alzheimer patient amyloid-B assembly, PNAS August 11, 2015 vol. 112 no. 32 E4465-E4474
[Non-Patent Document 3] Noguchi et al., Isolation and characterization of patient-derived, toxic, high mass amyloid beta-protein (Abeta) assembly from Alzheimer disease brains, J Biol Chem. 2009 Nov 20;284(47):32895-905

[SUMMARY OF THE INVENTION]

[Problems to be Solved by the Invention]

It is considered that if binding between ASPD and neurons can be inhibited, the interaction between ASPD and neurons can be inhibited, and thereby the neuronal cell death caused by ASPD can be avoided. Furthermore, it is considered that if a substance capable of inhibiting the binding between ASPD and neurons can be obtained, it can contribute to the development of treatment methods, for example, for Alzheimer's disease and Lewy body dementia.
Thus, in one aspect, the present disclosure provides a peptide capable of inhibiting binding between ASPD and neurons.

[Means for Solving Problem]

In one aspect, the present disclosure relates to a synthesized, isolated, or purified polypeptide, the polypeptide containing an amino acid sequence represented by Formula (I) below:

$$X_1X_2X_3X_4 \qquad (I)$$

(SEQ ID NO: 1),

where $X_1$ is R, H, or K,
$X_2$ is R, K, D, H, W, Q, Y, or T,
$X_3$ is any amino acid, and
$X_4$ is W, F, or Y.

In one aspect, the present disclosure relates to a synthesized, isolated, or purified polypeptide, the polypeptide containing an amino acid sequence represented by Formula (II) below:

$$X_1X_2X_3X_4 \qquad (II)$$

(SEQ ID NO: 2),

where $X_1$ is R, H, or K,
$X_2$ is any amino acid,
$X_3$ is L, G, I, W, R, M, D, E, A, V, or K, and
$X_4$ is W, F, or Y.

In one aspect, the present disclosure relates to a synthesized, isolated, or purified polypeptide, the polypeptide having an amino acid sequence represented by HANW, HVNW, HFHW, or HFYW.
In one aspect, the present disclosure relates to a peptide mimic having a structure mimicking the structure of a polypeptide according to the present disclosure, the peptide mimic having an ability to inhibit binding between ASPD and neurons.
In one aspect, the present disclosure relates to a vector for expressing a polypeptide according to the present disclosure, the vector containing a polynucleotide that encodes the polypeptide. In another aspect, the present disclosure relates to a composition containing a polypeptide according to the present disclosure or a peptide mimic according to the present disclosure.
In another aspect, the present disclosure relates to a method of evaluating a substance that inhibits binding between ASPD and neurons, the method including:

contacting a liquid mixture containing a substance to be evaluated and ASPD with cultured neurons or contacting the ASPD with the cultured neurons in the presence of the substance to be evaluated,
thereafter providing the ASPD and the neurons with different labels from each other to perform imaging, and
obtaining an indicator of binding of the ASPD to the neurons from data obtained by the imaging to evaluate the substance to be evaluated using the indicator.

In another aspect, the present disclosure relates to a method of screening a substance that inhibits neurotoxicity caused by ASPD, the method including:

contacting a liquid mixture containing a candidate substance and ASPD with cultured neurons or contacting the ASPD with the cultured neurons in the presence of the candidate substance,

thereafter providing the ASPD and the neurons with different labels from each other to perform imaging, and obtaining an indicator of binding of the ASPD to the neurons from data obtained by the imaging to select the candidate substance using the indicator.

[Brief Description of Drawings]

FIG. 1 shows examples of the results of fluorescence microscopic observation, with neurons and ASPD being immunofluorescence stained. FIG. 1 shows a comparison between when ASPD alone were contacted with neurons and when ASPD were pretreated with a given peptide, with the concentration of the ASPD to be added being changed.

FIG. 2 shows an example of a graph illustrating the relationship between the concentration of added ASPD and the amount of ASPD bound to neurons, which can be created from the results of microscopic observation.

FIG. 3 shows an example of a graph obtained by evaluating the ability, of peptides indicated on the horizontal axis, to inhibit binding between ASPD and neurons.

FIG. 4 shows an example of a graph obtained by evaluating the ability, of peptides indicated on the horizontal axis, to inhibit binding between ASPD and neurons.

FIG. 5 shows an example of a graph obtained by evaluating the ability, of peptides indicated on the horizontal axis, to inhibit binding between ASPD and neurons.

[Description of Preferred Embodiments]

In one aspect, the present disclosure relates to first and second peptides described below. That is, in one aspect, the present disclosure relates to a peptide having an amino acid sequence represented by Formula (I) or (II) below.

[First Peptide]

In one aspect, the present disclosure relates to a synthesized, isolated, or purified polypeptide (hereinafter also referred to as a "first peptide") containing an amino acid sequence represented by Formula (I) below:

$$X_1X_2X_3X_4 \qquad \text{(I)}$$

(SEQ ID NO: 1),

where $X_1$ is arginine (R), histidine (H), or lysine (K),

$X_2$ is arginine (R), aspartic acid (D), glutamine (Q), histidine (H), lysine (K), threonine (T), tryptophan (W), or tyrosine (Y),

$X_3$ is any amino acid, and

$X_4$ is tryptophan (W), phenylalanine (F), or tyrosine (Y).

In Formula (I), in one or more embodiments, $X_1$ is preferably R or H, more preferably H, from the viewpoint of inhibiting binding between ASPD and neurons.

In Formula (I), in one or more embodiments, from the same viewpoint, $X_2$ is:

preferably H, W, Q, Y, or T, and

more preferably H or T.

In Formula (I), in one or more embodiments, from the same viewpoint, $X_3$ is: preferably alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), proline (P), serine (S), threonine (T), tryptophan (W), tyrosine (Y), or valine (V), more preferably N, L, G, I, W, R, M, D, E, A, V, K, H, or Y,

further preferably N, L, I, W, R, D, K, H, or Y, and

further more preferably N, R, K, H, or Y

In Formula (I), in one or more embodiments, $X_4$ is preferably W from the same viewpoint.

One or more embodiments of the amino acid sequence represented by Formula (I) include the amino acid sequences of the peptides 02 to 11 indicated in Table 1 below, include HHNW, HWNW, HQNW, HYNW, and HTNW, or include HHNW and HTNW.

[Second Peptide]

In one aspect, the present disclosure relates to a synthesized, isolated, or purified polypeptide (hereinafter also referred to as a "second peptide") containing an amino acid sequence represented by Formula (II) below:

$$X_1X_2X_3X_4 \qquad \text{(II)}$$

(SEQ ID NO: 2),

where $X_1$ is arginine (R), histidine (H), or lysine (K),
$X_2$ is any amino acid,
$X_3$ is leucine (L), glycine (G), isoleucine (I), tryptophan (W), arginine (R), methionine (M), aspartic acid (D), glutamic acid (E), alanine (A), valine (V), or lysine (K), and $X_4$ is phenylalanine (F), tryptophan (W), or tyrosine (Y).

In Formula (II), in one or more embodiments, $X_1$ is preferably R or H, more preferably H, from the viewpoint of inhibiting binding between ASPD and neurons.
In Formula (II), in one or more embodiments, from the same viewpoint, $X_2$ is: preferably alanine (A), arginine (R), aspartic acid (D), glutamine (Q), glycine (G), histidine (H), isoleucine (I), leucine (L), lysine (K), methionine (M), phenylalanine (F), proline (P), threonine (T), tryptophan (W), tyrosine (Y), or valine (V),
more preferably F, R, K, D, H, W, Q, Y, T, A, or V,
further preferably F, H, W, Q, Y, T, A, or V, and
further more preferably F, H, T, A, or V.
In formula (II), in one or more embodiments, from the same viewpoint, $X_3$ is:

more preferably L, I, W, R, D, or K, and
further preferably R or K.

In the formula (II), in one or more embodiments, from the same viewpoint, $X_4$ is preferably W
One or more embodiments of the amino acid sequence represented by Formula (II) include the amino acid sequences of peptides 12 to 24 indicated in Table 2 below, include HFLW, HFIW, HFWW, HFRW, HFDW, and HFKW, or include HFRW and HFKW.

[Third Peptide]

In another aspect, the present disclosure relates to a synthesized, isolated, or purified polypeptide (hereinafter also referred to as a "third peptide") containing an amino acid sequence represented by HANW, HVNW, HFHW, or HFYW.
It is preferable that the first to third peptides according to the present disclosure can inhibit binding between ASPD and neurons in one or more embodiments.
It is preferable that the first to third peptides according to the present disclosure have an ability to inhibit the interaction between ASPD and NAKα3 in one or more embodiments.
It is preferable that the first to third peptides according to the present disclosure have an ability to bind to ASPD in one or more embodiments.
It is preferable that the first to third peptides according to the present disclosure can suppress ASPD-induced cell death of neurons in one or more embodiments.
In the present disclosure, when simply a term "amylospheroids (ASPD)" is used, it may include native ASPD and synthetic ASPD.
In the one or more embodiments, from the viewpoint of inhibiting binding between ASPD and neurons, it is preferable that the first to third peptides according to the present disclosure have, at its N terminus, an amino acid sequence represented by the Formula (I) or (II) or HANW, HVNW, HFHW, or HFYW, that is, the first amino acid at the N terminus is $X_1$ or H.
In one or more embodiments, the lengths of the first to third peptides according to the present disclosure can be, for example, 4 or more but 50 or less, 25 or less, 20 or less, 15 or less, 10 or less, or 5 or less. Therefore, examples of the lengths of the peptides include 4 to 50, 4 to 25, 4 to 20, 4 to 15, 4 to 10, or 4 to 5.
In one or more embodiments, the first to third peptides according to the present disclosure can be in the form in which an amino acid sequence represented by the Formula (I) or (II) or HANW, HVNW, HFHW, or HFYW and a brain-migrating peptide are bound to each other directly or through a linker peptide. Binding a brain-migrating peptide can increase the efficiency of migration of the polypeptide of the present disclosure across the blood brain barrier into the brain. For the brain-migrating peptide, conventionally known ones can be used.

The first to third peptides according to the present disclosure can also include those in the forms in which said peptides have been modified. In one or more embodiments, the forms in which the terminals of said peptides and/or peptide bonds have been modified can also be included. Examples of the terminal modification include removal or amidation of the C-terminal carboxyl group and removal or acetylation of the N-terminal amino group. Examples of the modification of the peptide bonds include N-alkylation.

In one or more embodiments, from the viewpoint of inhibiting binding between ASPD and neurons, in the first to third peptides according to the present disclosure, the C-terminal carboxyl group preferably has been removed or amidated and more preferably has been removed. Examples of the amidation of the C-terminal carboxyl group include -$CONH_2$, -CONHR, and -CONRR' (R and R' are hydrocarbon groups), and preferably it is -$CONH_2$.

The N-terminal amino group may be left as it is or may be acetylated or removed, but preferably it is left as it is from the viewpoint of inhibiting binding between ASPD and neurons.

In one or more embodiments, with respect to the first to third peptides according to the present disclosure, from the viewpoint of promoting the binding of said peptides to ASPD, at least one of the peptide bonds may have been N-alkylated. Examples of N-alkylation include N-methylation, N-ethylation, and N-cyclopropylation.

The first to third peptides according to the present disclosure can be prepared by appropriate synthesis according to an ordinary synthesis method, and purification.

[Peptide Mimic]

In another aspect, the present disclosure relates to peptide mimics having structures mimicking the structures of the first to third peptides according to the present disclosure.

One or more embodiments of the "peptide mimic" in the present disclosure include peptide-like compounds or synthetic compounds that do not have the structure of a peptide bond and the structure of a modified peptide bond.

One or more embodiments of the peptide-like compounds include, for example, peptoid (S. M. Miller, R. J. Simon, S. Ng, R. N. Zuckermann, J. S. Kerr, W. H. Moos, Bioorg. Med. Chem Lett., 4, 2657 (1994)) and nonpeptide compounds mimicking the $\beta/\gamma$ turn structure of proteins (M. Kahn Tetrahedron, 49, 3433 (1993), "Combinatorial Chemistry" published by Kagaku Dojin, p44-64, 1997).

In one or more embodiments, it is preferable that the peptide mimic according to the present disclosure can inhibit binding between ASPD and neurons. In one or more embodiments, the peptide mimic according to the present disclosure preferably has an ability to inhibit the interaction between ASPD and $NAK\alpha3$. In one or more embodiments, the peptide mimic according to the present disclosure preferably has an ability to bind to ASPD. In one or more embodiments, it is preferable that the peptide mimic according to the present disclosure can suppress ASPD-induced cell death of neurons.

[Polynucleotide and Vector]

In another aspect, the present disclosure relates to a polynucleotide that encodes the polypeptides of the present disclosure, and to a vector that contains a polynucleotide that encodes the polypeptides of the present disclosure and is used for expressing the above-mentioned polypeptides. Preferably, the vector has an expression cassette containing, for example, a regulatory sequence that enables the expression of the polynucleotide of the present disclosure. The vector is not particularly limited as long as it allows gene transfer of the polynucleotide, but an AW (adeno-associated virus) vector is preferred from the viewpoint of safety. Preferred embodiments of the vector of the present disclosure include those having a form in which the vector is bound to a brain-migrating peptide. Binding of a brain-migrating peptide to the vector can increase the efficiency of migration of the vector of the present disclosure across the blood brain barrier into the brain. For the brain-migrating peptide, conventionally known ones can be used.

[Composition]

As another aspect, the present disclosure relates to a composition containing a peptide according to the present disclosure, a peptide mimic according to the present disclosure, or a vector according to the present disclosure. In one or more embodiments, the above-mentioned composition is a pharmaceutical composition.

[Pharmaceutical Composition]

When the present disclosure is a pharmaceutical composition, the dosage form thereof can be suitably selected according to the administration method, and examples thereof include injections, liquid formulations, capsules, chews, tablets, suspensions, creams, and ointments. Furthermore, the method of administration is also not particularly limited and examples thereof include oral administration and parenteral administration. The pharmaceutical composition of the present disclosure may contain conventionally known additives (for example, excipients or diluents) depending on the administration form and dosage form.

Examples of the dosage form suitable for oral administration include; solid formulations such as tablets, particles, liquid- or powder-containing capsules, troches, chews, multiparticles and nanoparticles, gels, and films; and liquid formulations such as suspensions, solutions, syrups, and elixirs. Examples of the excipient include carriers such as cellulose, calcium carbonate, dicalcium phosphate, mannitol, and sodium citrate; granulating binders such as polyvinylpyrrolidine, hydroxypropyl cellulose, hydroxypropyl methylcellulose, and gelatin; disintegrators such as sodium starch glycolate and silicate; lubricants such as magnesium stearate and stearic acid; humectants such as sodium lauryl sulfate; preservatives, antioxidants, flavoring agents, and colorants.

Examples of parenteral administration of the pharmaceutical composition of the present disclosure include direct administration into a bloodstream, a muscle, or an internal organ. Examples of parenteral administration include intravenous, intraarterial, intraperitoneal, intrathecal, intraventricular, intraurethral, intrasternal, intracranial, intramuscular, and subcutaneous administrations. Parenteral administration can be performed, for example, with a needle syringe, a needleless syringe, and other injection techniques. Furthermore, examples of a dosage form suitable for parenteral administration include an aqueous solution containing an excipient and/or a buffer agent.

[Prevention, Amelioration, and/or Treatment Method]

In one or more embodiments, the pharmaceutical composition according to the present disclosure can be used for the prevention, amelioration, and/or treatment of Alzheimer's disease and/or Lewy body dementia.

In the present disclosure, the prevention of Alzheimer's disease and/or Lewy body dementia refers to the inhibition of the development of Alzheimer's disease and/or Lewy body dementia or the prevention of the progression of the pathological condition of reversible mild cognitive impairment. Furthermore, in the present disclosure, the amelioration of Alzheimer's disease and/or Lewy body dementia includes stopping the progression of the pathological condition or improving the pathological condition of reversible mild cognitive impairment of Alzheimer's disease and/or Lewy body dementia. Furthermore, in the present disclosure, the treatment of Alzheimer's disease and/or Lewy body dementia includes delaying or almost stopping the progression of the pathological condition.

[Evaluation Method]

In another aspect, the present disclosure relates to a method of evaluating a substance that inhibits binding between ASPD and neurons.

A first evaluation method may include contacting a liquid mixture containing a substance to be evaluated and ASPD with cultured neurons, thereafter providing the ASPD and the neurons with different labels from each other to perform imaging, and obtaining an indicator of the amount of the ASPD bound to the neurons from data obtained by the imaging to evaluate the substance to be evaluated using the indicator. A second evaluation method may include contacting ASPD with cultured neurons in the presence of a substance to be evaluated, thereafter providing the ASPD and the neurons with different labels from each other to perform imaging, and obtaining an indicator of the amount of the ASPD bound to the neurons from data obtained by the imaging to evaluate the substance to be evaluated using the indicator.

According to the first evaluation method, it is possible to evaluate the binding inhibitory ability, with respect to the binding between ASPD and neurons, of a substance that binds to or interacts with ASPD. According to the second evaluation method, it is possible to evaluate the binding inhibitory ability, with respect to the binding between ASPD and neurons, of a substance that binds to or interacts with neurons.

Preferably, the neurons are mature neurons. In one or more embodiments, mature neurons can be those having at least one axon and at least one dendrite.

In one or more embodiments, examples of the imaging method include immunofluorescence staining and fluorescence microscopic observation. In one or more embodiments, the neurons and the ASPD may be immobilized after the contacting but before the labeling.

In this aspect, when calculating the amount of the ASPD bound or the indicator thereof, it is easy to eliminate, from image data, information on large aggregates of APSD and/or information on nonspecific ASPD not bound to neurons. This makes it possible to improve the quantitativeness and/or the sensitivity of the evaluation in one or more embodiments.

In one or more embodiments, the indicator of the amount of ASPD bound may be the amount itself of the ASPD bound to neurons or may be $ED_{50}$ or the ratio thereof as described in the Examples. For example, it can be evaluated that the higher the $ED_{50}$ in the case where the substance to be evaluated and ASPD coexist, the higher the ability of inhibiting ASPD from binding to neurons.

[Screening Method]

In another aspect, the present disclosure relates to a method of screening a substance that inhibits neurotoxicity caused by ASPD.

A first screening method may include contacting a liquid mixture containing a candidate substance and ASPD with cultured neurons, thereafter providing the ASPD and the neurons with different labels from each other to perform imaging, and obtaining an indicator of binding of the ASPD to the neurons from data obtained by the imaging to select a candidate substance using the indicator.

A second screening method may include contacting ASPD with cultured neurons in the presence of a candidate substance, thereafter providing the ASPD and the neurons with different labels from each other to perform imaging, and obtaining an indicator of binding of the ASPD to the neurons from data obtained by the imaging to select a candidate substance using the indicator.

According to the first screening method, a substance that binds to or interacts with ASPD and has an ability to inhibit binding between ASPD and neurons can be selected as a candidate substance for a substance that inhibits neurotoxicity caused by ASPD.

According to the second screening method, a substance that binds to or interacts with neurons and has an ability to inhibit binding between ASPD and neurons can be selected as a candidate substance for a substance that inhibits neurotoxicity caused by ASPD.

The neurons and the imaging method can be the same as those used for the evaluation method according to the present disclosure. Furthermore, in one or more embodiments, the indicator of the amount of ASPD bound may be the amount itself of the ASPD bound to neurons or may be $ED_{50}$ or the ratio thereof. For example, it can be evaluated that the higher the $ED_{50}$ in the case where the candidate substance and ASPD coexist, the higher the ability of inhibiting neurotoxicity of ASPD.

In this aspect, when calculating the amount of the ASPD bound or the indicator thereof, it is easy to eliminate, from image data, information on large aggregates of APSD and/or information on nonspecific ASPD not bound to neurons. This makes it possible to improve the quantitativeness and/or the screening sensitivity in one or more embodiments.

In one or more embodiments, the present disclosure may relate to the following:

[1] A synthesized, isolated, or purified polypeptide,
the polypeptide containing an amino acid sequence represented by Formula (I) below:

$$X_1X_2X_3X_4 \qquad \text{(I)}$$

(SEQ ID NO: 1)
where $X_1$ is arginine (Arg), histidine (His), or lysine (Lys),
$X_2$ is arginine (Arg), lysine (Lys), aspartic acid (Asp), histidine (His), tryptophan (Trp), glutamine (Gln), tyrosine (Tyr), or threonine (Thr),
$X_3$ is any amino acid, and
$X_4$ is tryptophan (Trp), phenylalanine (Phe), or tyrosine (Tyr).

[2] The polypeptide according to [1], wherein $X_3$ is alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), proline (Pro), serine (Ser), threonine (Thr), tryptophan (Trp), tyrosine (Tyr), or valine (Val).

[3] The polypeptide according to [1] or [2], wherein $X_2$ is histidine (His), tryptophan (Trp), glutamine (Gln), tyrosine (Tyr), or threonine (Thr).

[4] A synthesized, isolated, or purified polypeptide,
the polypeptide containing an amino acid sequence represented by Formula (II) below:

$$X_1X_2X_3X_4 \qquad \text{(II)}$$

(SEQ ID NO: 2),
where $X_1$ is arginine (Arg), histidine (His), or lysine (Lys),
$X_2$ is any amino acid,
$X_3$ is leucine (Leu), glycine (Gly), isoleucine (Ile), tryptophan (Trp), arginine (Arg), methionine (Met), aspartic acid (Asp), glutamic acid (Glu), alanine (Ala), valine (Val), or lysine (Lys), and
$X_4$ is tryptophan (Trp), phenylalanine (Phe), or tyrosine (Tyr).

[5] The polypeptide according to [4], wherein $X_2$ is alanine (Ala), arginine (Arg), aspartic acid (Asp), glutamine (Gln), glycine (Gly), histidine (His), isoleucine (Ile), leucine (Leu), lysine (Lys), methionine (Met), phenylalanine (Phe), proline (Pro), threonine (Thr), tryptophan (Trp), tyrosine (Tyr), or valine (Val).

[6] The polypeptide according to [4] or [5], wherein $X_3$ is leucine (Leu), isoleucine (Ile), tryptophan (Trp), arginine (Arg), aspartic acid (Asp), or lysine (Lys).

[7] A synthesized, isolated, or purified polypeptide, the polypeptide having an amino acid sequence represented by HANW, HVNW, HFHW, or HFYW.

[8] The polypeptide according to any one of [1] to [7], the polypeptide having, at its N terminus, an amino acid sequence represented by the Formula (I) or (II), or HANW, HVNW, HFHW, or HFYW.

[9] The polypeptide according to any one of [1] to [8], wherein the amino acid sequence has a length of 4 to 50 amino acids.

[10] The polypeptide according to any one of [1] to [9], wherein its C-terminal carboxyl group has been removed or amidated.

[11] The polypeptide according to any one of [1] to [10], wherein at least one of peptide bonds has been N-alkylated.

[12] The polypeptide according to any one of [1] to [11], wherein the polypeptide has an ability to inhibit binding between ASPD and neurons.

[13] A peptide mimic having a structure mimicking the structure of a polypeptide according to any one of [1] to [12], the peptide mimic having an ability to inhibit binding between ASPD and neurons.

[14] A vector for expressing a polypeptide according to any one of [1] to [12], the vector containing a polynucleotide that encodes the polypeptide.

[15] A composition containing a polypeptide according to any one of [1] to [12] or a peptide mimic according to [13].

[16] A method of evaluating a substance that inhibits binding between amylospheroids (ASPD) and neurons, the method including:

contacting a liquid mixture containing a substance to be evaluated and ASPD with cultured neurons, or contacting ASPD with cultured neurons in the presence of the substance to be evaluated,

thereafter providing the ASPD and the neurons with different labels from each other to perform imaging, and obtaining an indicator of binding of the ASPD to the neurons from data obtained by the imaging to evaluate the substance to be evaluated using the indicator.

[17] A method of screening a substance that inhibits neurotoxicity caused by amylospheroids (ASPD), the method including:

contacting a liquid mixture containing a candidate substance and ASPD with cultured neurons or contacting the ASPD with the cultured neurons in the presence of the candidate substance,

thereafter providing the ASPD and the neurons with different labels from each other to perform imaging, and obtaining an indicator of binding of the ASPD to the neurons from the data obtained by the imaging to select a candidate substance using the indicator.

Hereinafter, one or more embodiments of the present invention will be further described using examples.

[Examples]

1. Evaluation of ASPD-inhibitory peptides

Evaluation of peptides that inhibits binding of ASPD to neurons was performed by the following method.

Step 1: Adding a liquid mixture containing peptide and ASPD, which have been mixed together and then incubated, to cultured mature neurons.
Step 2: Degenerating (immobilizing) the neurons.
Step 3: Immunostaining the neurons and the ASPD, respectively, to perform imaging.
Step 4: Analyzing the image to quantify the ASPD bound to the neurons.
Step 5: Evaluating the peptides using the amount of the ASPD bound and/or an analytical value obtained therefrom.

Steps 1 to 3 were performed as follows:
A liquid mixture of peptide and synthetic ASPD was incubated at 4°C for 15 minutes to pretreat the ASPD and this liquid mixture was added to primary cultured rat hippocampal neurons cultured in a 96-well plate.

After incubation at 37°C for 15 minutes in a $CO_2$ incubator, the wells were washed with PBS. Then, 2% para-formaldehyde (50 μl/well) was added thereto for immobilization performed for 20 minutes at 37°C and further 4% para-formaldehyde (50 μl/well) was added thereto for immobilization performed for 20 minutes at 37°C. After washing and blocking, a primary antibody solution containing anti-ASPD antibody (mASD3) and anti-MAP2 antibody (trade name: sc-20172, manufactured by SANTACRUZ) was added, which then was left overnight. After washing, a secondary antibody solution containing Alexa Fluoro (trademark) 568 anti-mouse antibody (trade name: A11031, manufactured by life technologies), Alexa Fluoro (trademark) 488 anti-rabbit antibody (trade name: A11034, manufactured by life technologies), and DAPI (trade name: D523, manufactured by Dojindo) was added and then fluorescence microscopic observation was performed.

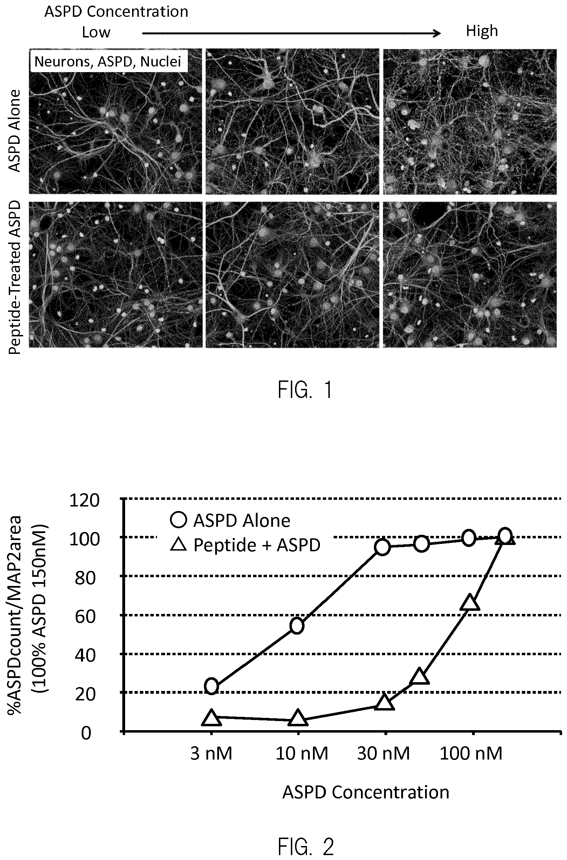

Examples of the results of the images obtained by microscopic observation are shown in FIG. 1. FIG. 1 shows a comparison between when ASPD alone were contacted with neurons and when ASPD were pretreated with peptide, with the concentration of the ASPD to be added being changed. In FIG. 1, the large round shapes (blue) are nuclei, the elongated shapes (green) are neuronal axons and dendrites, and the small round shapes (red) are the ASPD. As shown in the figure, in the case of the ASPD alone, the amount of the ASPD around the neurons increased with an increase in the concentration of the ASPD added, whereas in the case of the ASPD treated with peptide, the amount of the ASPD did not increase with an increase in the concentration of the ASPD added. That is, it showed that the peptide had inhibited the binding between the ASPD and the neurons.

Subsequently, steps 4 to 5 were performed as follows:

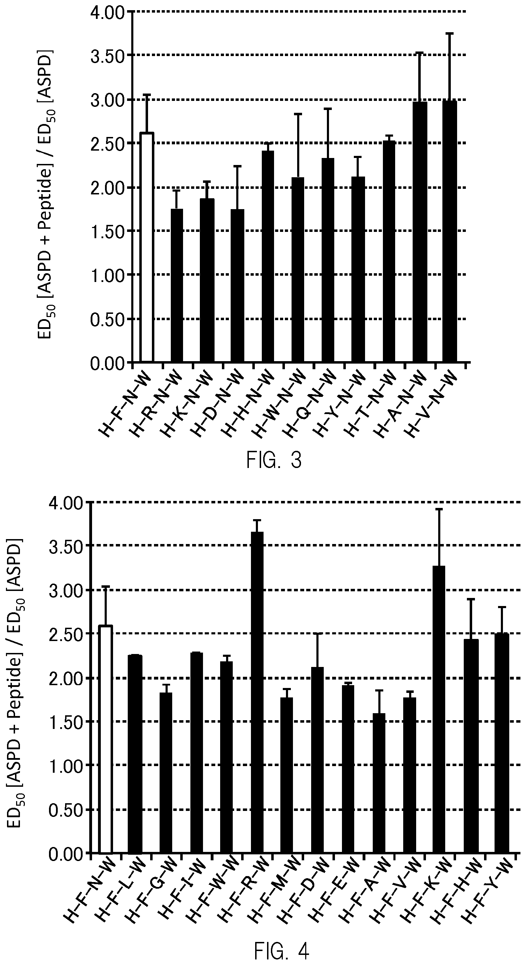

From the microscopic observation data, a calibration curve as shown in FIG. 2 was drawn, an approximate curve was created within a range where linearity was maintained, and then $ED_{50}$ was calculated. Using, as an indicator, "titer" that is defined by the ratio of $ED_{50}$ of the ASPD treated with peptide to $ED_{50}$ of the ASPD alone, the ASPD-binding inhibitory ability of peptide was evaluated. The results of peptides 01 to 11 shown in Table 1 are shown in FIG. 3. The results of peptides 01 and 12 to 24 shown in Table 2 are shown in FIG. 4.

$$\text{Titer} = ED_{50} \, [\text{ASPD} + \text{Peptide}] \, / \, ED_{50} \, [\text{ASPD}]$$

[Table 1]

| Table 1 | X1 | X2 | X3 | X4 | |
|---|---|---|---|---|---|
| Peptide 01 | H | F | N | W | (SEQ ID NO: 3) |
| Peptide 02 | H | R | N | W | (SEQ ID NO: 4) |
| Peptide 03 | H | K | N | W | (SEQ ID NO: 5) |
| Peptide 04 | H | D | N | W | (SEQ ID NO: 6) |
| Peptide 05 | H | H | N | W | (SEQ ID NO: 7) |
| Peptide 06 | H | W | N | W | (SEQ ID NO: 8) |
| Peptide 07 | H | Q | N | W | (SEQ ID NO: 9) |
| Peptide 08 | H | Y | N | W | (SEQ ID NO: 10) |
| Peptide 09 | H | T | N | W | (SEQ ID NO: 11) |
| Peptide 10 | H | A | N | W | (SEQ ID NO: 12) |
| Peptide 11 | H | V | N | W | (SEQ ID NO: 13) |

[Table 2]

| Table 2 | X1 | X2 | X3 | X4 | |
|---|---|---|---|---|---|
| Peptide 01 | H | F | N | W | (SEQ ID NO: 3) |
| Peptide 12 | H | F | L | W | (SEQ ID NO: 14) |
| Peptide 13 | H | F | G | W | (SEQ ID NO: 15) |
| Peptide 14 | H | F | I | W | (SEQ ID NO: 16) |
| Peptide 15 | H | F | W | W | (SEQ ID NO: 17) |
| Peptide 16 | H | F | R | W | (SEQ ID NO: 18) |

(continued)

| Table 2 | X1 | X2 | X3 | X4 | |
|---|---|---|---|---|---|
| Peptide 17 | H | F | M | W | (SEQ ID NO: 19) |
| Peptide 18 | H | F | D | W | (SEQ ID NO: 20) |
| Peptide 19 | H | F | E | W | (SEQ ID NO: 21) |
| Peptide 20 | H | F | A | W | (SEQ ID NO: 22) |
| Peptide 21 | H | F | V | W | (SEQ ID NO: 23) |
| Peptide 22 | H | F | K | W | (SEQ ID NO: 24) |
| Peptide 23 | H | F | H | W | (SEQ ID NO: 25) |
| Peptide 24 | H | F | Y | W | (SEQ ID NO: 26) |

As shown in FIG. 3, the peptides 02 to 11 each showed an ASPD-binding inhibitory ability (titer) of 1.5 or more. Among them, the peptides 05 (HHNW), 09 (HTNW), 10 (HANW), and 11 (HVNW) showed an ASPD-binding inhibitory ability (titer) comparable to that of known ASPD neutralizing peptide 01 (HFNW).

In addition, as shown in FIG. 4, the peptides 12 to 24 each showed an ASPD-binding inhibitory ability (titer) of 1.5 or more. Among them, the peptide 16 (HFRW) and the peptide 22 (HFKW) showed a superior ASPD-binding inhibition ability (titer) as compared to that of the known ASPD neutralizing peptide 01 (HFNW).

2. Surface Plasmon Resonance (SPR)

Synthetic ASPD were solid-phased on a chip and each peptide shown in Table 3 below was allowed to flow as an analyte. Then the dissociation constant ($K_D$) was determined by surface plasmon resonance (SPR).

The peptides used herein are the peptides 01, 05, 08, 09, 15, and 18 shown in FIGS. 3 and 4, and all of them showed the effect of inhibiting binding of ASPD to neurons. Two peptides (HNNW (SEQ ID NO: 27) and HPNW (SEQ ID NO: 28)) that could not inhibit the binding of ASPD to neurons in the evaluation performed in section 1 above were used as negative controls.

The results are shown in Table 3 below.

[Table 3]

| Table 3 | Sequence | Results of SPR (Ability to Bind to ASPD) |
|---|---|---|
| Peptide 01 | HFNW | + |
| Peptide 05 | HHNW | + |
| Peptide 08 | HYNW | ++ |
| Peptide 09 | HTNW | +++ |
| Peptide 15 | HFWW | + |
| Peptide 18 | HFDW | + |
| Negative Control | HNNW | No binding was found. |
| Negative Control | HPNW | No binding was found. |

As shown in Table 3 above, it was shown that peptides having an effect of inhibiting the binding of ASPD to neurons in the evaluation performed in section 1 above had an ability to bind to ASPD.

3. Treatment of Peptide Terminus

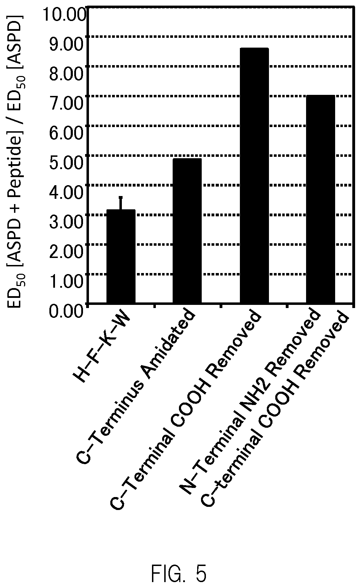

The ASPD-binding inhibitory ability (titer) was calculated in the same manner as described above using peptides obtained by amidating ($-CONH_2$) or removing the COOH at the C-terminus of the peptide 22 (HFKW) and a peptide from which the amino group at the N-terminus was removed. The results are shown in FIG. 5. As shown in FIG. 5, it was shown that when the C terminus was treated (amidation or removal of COOH), the ASPD-inhibiting ability of the peptides was improved. Furthermore, even in the case of using peptides other than the peptide 22, it was shown that when the C-terminus was treated (amidation or removal of COOH), the ASPD-inhibiting ability of the peptides tended to be improved.

4. N-Methylation of Peptide Bond

One or two of the peptide bonds of the peptide 22 (HFKW) were N-methylated, and the dissociation constant ($K_D$) was determined by surface plasmon resonance (SPR) in the same manner as in section 2 described above. As a result, each of the N-methylated peptides 22 was found to have an ability to bind to ASPD.

**Claims**

1.  A synthesized, isolated, or purified polypeptide,
    comprising an amino acid sequence represented by Formula (I) below:

    $$X_1X_2X_3X_4 \qquad (I)$$

    (SEQ ID NO: 1),

    where $X_1$ is arginine (Arg), histidine (His), or lysine (Lys),
    $X_2$ is arginine (Arg), lysine (Lys), aspartic acid (Asp), histidine (His), tryptophan (Trp), glutamine (Gln), tyrosine (Tyr), or threonine (Thr),
    $X_3$ is any amino acid, and
    $X_4$ is tryptophan (Trp), phenylalanine (Phe), or tyrosine (Tyr).

2.  The polypeptide according to claim 1, wherein $X_3$ is alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), proline (Pro), serine (Ser), threonine (Thr), tryptophan (Trp), tyrosine (Tyr), or valine (Val).

3.  The polypeptide according to claim 1 or 2, wherein $X_2$ is histidine (His), tryptophan (Trp), glutamine (Gln), tyrosine (Tyr), or threonine (Thr).

4.  A synthesized, isolated, or purified polypeptide,
    comprising an amino acid sequence represented by Formula (II) below:

    $$X_1X_2X_3X_4 \qquad (II)$$

    (SEQ ID NO: 2),

    where $X_1$ is arginine (Arg), histidine (His), or lysine (Lys),
    $X_2$ is any amino acid,
    $X_3$ is leucine (Leu), glycine (Gly), isoleucine (Ile), tryptophan (Trp), arginine (Arg), methionine (Met), aspartic acid (Asp), glutamic acid (Glu), alanine (Ala), valine (Val), or lysine (Lys), and
    $X_4$ is tryptophan (Trp), phenylalanine (Phe), or tyrosine (Tyr).

5.  The polypeptide according to claim 4, wherein $X_2$ is alanine (Ala), arginine (Arg), aspartic acid (Asp), glutamine (Gln), glycine (Gly), histidine (His), isoleucine (Ile), leucine (Leu), lysine (Lys), methionine (Met), phenylalanine (Phe), proline (Pro), threonine (Thr), tryptophan (Trp), tyrosine (Tyr), or valine (Val).

6.  The polypeptide according to claim 4 or 5, wherein $X_3$ is leucine (Leu), isoleucine (Ile), tryptophan (Trp), arginine (Arg), aspartic acid (Asp), or lysine (Lys).

7.  A synthesized, isolated, or purified polypeptide, comprising an amino acid sequence represented by HANW, HVNW, HFHW, or HFYW.

8.  The polypeptide according to any one of claims 1 to 7, wherein the polypeptide comprising, at its N terminus, an amino acid sequence represented by the Formula (I) or (II), or HANW, HVNW, HFHW, or HFYW.

9. The polypeptide according to any one of claims 1 to 8, wherein the amino acid sequence has a length of 4 to 50 amino acids.

10. The polypeptide according to any one of claims 1 to 9, wherein its C-terminal carboxyl group has been removed or amidated.

11. The polypeptide according to any one of claims 1 to 10, wherein at least one of peptide bonds has been N-alkylated.

12. The polypeptide according to any one of claims 1 to 11, wherein the polypeptide has an ability to inhibit binding between ASPD and a neuron.

13. A peptide mimic, comprising a structure mimicking a structure of a polypeptide according to any one of claims 1 to 12, the peptide mimic having an ability to inhibit binding between ASPD and a neuron.

14. A vector for expressing a polypeptide according to any one of claims 1 to 12, the vector comprising a polynucleotide that encodes the polypeptide.

15. A composition, comprising a polypeptide according to any one of claims 1 to 12 or a peptide mimic according to claim 13.

16. A method of evaluating a substance that inhibits binding between amylospheroids (ASPD) and a neuron, the method comprising:

contacting a liquid mixture containing a substance to be evaluated and ASPD with a cultured neuron, or contacting ASPD with a cultured neuron in the presence of the substance to be evaluated,
thereafter providing the ASPD and the neuron with different labels from each other to perform imaging, and obtaining an indicator of binding of the ASPD to the neuron from data obtained by the imaging to evaluate the substance to be evaluated using the indicator.

17. A method of screening a substance that inhibits neurotoxicity caused by amylospheroids (ASPD), the method comprising:

contacting a liquid mixture containing a candidate substance and ASPD with a cultured neuron or contacting the ASPD with the cultured neuron in the presence of the candidate substance,
thereafter providing the ASPD and the neuron with different labels from each other to perform imaging, and obtaining an indicator of binding of the ASPD to the neuron from data obtained by the imaging to select a candidate substance using the indicator.

ASPD Concentration

Low ⟶ High

FIG. 1

FIG. 2

FIG. 3

FIG. 4

FIG. 5

## INTERNATIONAL SEARCH REPORT

International application No.

PCT/JP2017/015090

### A. CLASSIFICATION OF SUBJECT MATTER

*C07K5/11*(2006.01)i, *A61K38/07*(2006.01)i, *A61P25/28*(2006.01)i, *C07K5/00*
(2006.01)i, *C07K5/117*(2006.01)i, *C12N15/00*(2006.01)i, *C12N15/09*(2006.01)i,
*C12Q1/02*(2006.01)i, *G01N33/15*(2006.01)i, *G01N33/50*(2006.01)i

According to International Patent Classification (IPC) or to both national classification and IPC

### B. FIELDS SEARCHED

Minimum documentation searched (classification system followed by classification symbols)

C07K5/11, A61K38/07, A61P25/28, C07K5/00, C07K5/117, C12N15/00, C12N15/09,
C12Q1/02, G01N33/15, G01N33/50

Documentation searched other than minimum documentation to the extent that such documents are included in the fields searched

Jitsuyo Shinan Koho          1922-1996   Jitsuyo Shinan Toroku Koho   1996-2017
Kokai Jitsuyo Shinan Koho    1971-2017   Toroku Jitsuyo Shinan Koho   1994-2017

Electronic data base consulted during the international search (name of data base and, where practicable, search terms used)

CAplus/MEDLINE/EMBASE/BIOSIS(STN), JSTPlus/JMEDPlus/JST7580(JDreamIII),
UniProt/GeneSeq, PubMed

### C. DOCUMENTS CONSIDERED TO BE RELEVANT

| Category* | Citation of document, with indication, where appropriate, of the relevant passages | Relevant to claim No. |
|---|---|---|
| X/Y | WO 2013/099806 A1  (TAO Health Life Pharma Co., Ltd.), 04 July 2013 (04.07.2013), SEQ ID NO.7; paragraphs [0023], [0082] to [0092], [0025], [0059]; claim 12; paragraph [0073] & US 2013/0171069 A1 SEQ ID NO.7; paragraphs [0103], [0183] to [0192], [0106], [0154]; claim 12; paragraph [0175] & JP 2015-214557 A      & EP 2799542 A1 | 1-9,12-17/ 10,11 |
| Y | WO 2014/167122 A1  (BICYCLE THERAPEUTICS LTD.), 16 October 2014 (16.10.2014), page 25, line 4 to page 26, line 13 & JP 2016-519683 A     & US 2016/0046673 A1 & EP 2983722 A1 | 1-17 |

| ☒ | Further documents are listed in the continuation of Box C. | ☐ | See patent family annex. |
|---|---|---|---|

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 19 May 2017 (19.05.17) | 06 June 2017 (06.06.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2017/015090

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2013-509389 A  (Lytix Biopharma AS),<br>14 March 2013 (14.03.2013),<br>claim 8; paragraph [0032]<br>& WO 2011/051692 A1<br>claim 8; page 6, 4th paragraph<br>& US 2013/0035296 A1    & EP 2496596 A1 | 1-17 |
| Y | JP 2010-500298 A  (Carriero, Maria Vincenza et al.),<br>07 January 2010 (07.01.2010),<br>paragraphs [0024] to [0025]<br>& WO 2008/017372 A1<br>page 7, 6th to 7th paragraphs<br>& US 2011/0230397 A1    & EP 2049562 A1<br>& EP 2213680 A1          & EP 2230245 A1 | 1-17 |
| A | OHNISHI, T., et al., Na, K-ATPase α3 is a death target of Alzheimer patient amyloid-β assembly, 2015, Proceedings of the National Academy of Sciences of the United States of America, Vol. 112, No.32, E4465-4474 entire text | 1-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006016644 A
- WO 2009057664 A
- WO 2013099806 A
- WO 2013094614 A

### Non-patent literature cited in the description

- **HOSHI et al.** Spherical aggregates of β-amyloid (amylospheroids) show high neurotoxicity and activate tau protein kinase I/glycogen synthase kinase-3β. *PNAS,* 27 May 2003, vol. 100 (11), 6370-6375
- **OHNISHI et al.** Na, K-ATPase α3 is a death target of Alzheimer patient amyloid-B assembly. *PNAS,* 11 August 2015, vol. 112 (32), E4465-E4474
- **NOGUCHI et al.** Isolation and characterization of patient-derived, toxic, high mass amyloid beta-protein (Abeta) assembly from Alzheimer disease brains. *J Biol Chem.,* 20 November 2009, vol. 284 (47), 32895-905
- **S. M. MILLER ; R. J. SIMON ; S. NG ; R. N. ZUCKERMANN ; J. S. KERR ; W. H. MOOS.** *Bioorg. Med. Chem Lett.,* 1994, vol. 4, 2657
- **M. KAHN.** Tetrahedron. 1993, vol. 49, 3433
- Combinatorial Chemistry. Kagaku Dojin, 1997, 44-64